(19) Europäisches Patentamt
European Patent Office
Office européen des brevets

(11) Publication number: 0 088 974
A2

(12) # EUROPEAN PATENT APPLICATION

(21) Application number: 83102200.9

(22) Date of filing: 07.03.83

(51) Int. Cl.³: G 01 N 33/54

(30) Priority: 18.03.82 US 359610

(43) Date of publication of application:
21.09.83 Bulletin 83/38

(84) Designated Contracting States:
AT BE CH DE FR GB IT LI LU NL SE

(71) Applicant: MILES LABORATORIES INC.
1127 Myrtle Street
Elkhart Indiana 46514(US)

(72) Inventor: Burd, John F.
1000 Prairie Avenue
Elkhart, IN 46514(US)

(74) Representative: Senftl, Hannes, Dr. et al,
c/o Bayer AG Zentralbereich Patente Marken und Lizenzen
D-5090 Leverkusen 1, Bayerwerk(DE)

(54) **Homogeneous immunoassay with labelled monoclonal anti-analyte.**

(57) A homogeneous immunoassay method for determining an analyte, particularly analytes of molecular weight greater than about 10,000 (e.g., proteins), wherein sample is combined with a labeled monoclonal anti-analyte preparation in which the anti-analyte is substantially the only labeled component. Binding of the analyte with labeled anti-analyte results directly in a change in the detectable response generated by the label, usually due to steric constraints. The method provides a simple homogeneous technique which does not require the preparation of purified analyte in order to prepare assay reagents. Alternatively, the sample is combined with the labeled monoclonal anti-analyte and with a macromolecular conjugate having analyte epitope sites and binding of the labeled anti-analyte to such conjugate results in a change in the response of the label.

EP 0 088 974 A2

Croydon Printing Company Ltd.

# HOMOGENEOUS IMMUNOASSAY WITH LABELED MONOCLONAL ANTI-ANALYTE

## BACKGROUND OF THE INVENTION

### *1. FIELD OF THE INVENTION*

The development of immunoassay techniques has provided extremely useful analytical methods for determining various organic substances of diagnostic, medical, environmental and industrial importance which appear in liquid mediums at very low concentrations. Immunoassays are based on the specific immunological binding interaction between the substance under determination, herein referred to as analyte, e.g., an antigen, hapten or antibody, and its binding partner thereof, e.g., the corresponding antibody or antigen.

In conventional immunoassay techniques, a test sample to be assayed is combined in a liquid reaction mixture with reagent systems of various compositions. Such systems usually comprise (i) a labeled conjugate, which is a conjugate of the analyte, or a specific binding analog thereof, and a labeling substance, and (ii) a limiting amount of an antibody (anti-analyte) for the analyte. In the reaction mixture then, analyte in the sample and labeled conjugate compete for binding to the anti-analyte resulting in formation

of an antibody-bound-species and a free-species of the labeled conjugate. The relative amount or proportion of the labeled conjugate that results in the bound-species compared to the free-species is a function of the presence (or amount) of the analyte in the test sample. One can thus measure the labeling substance in the free- or bound-species and correlate the measured amount with the presence or amount of the analyte in the sample.

The first immunoassay to be developed was the radioimmunoassay which employs a radioactive isotope as the label. Because of the inconvenience and difficulty of handling radioactive materials, assay systems have been devised using materials other than radioisotopes as the label component, including enzyme cofactors, enzyme substrates, enzyme modulators, e.g., activators and inhibitors, cycling reactants, spin radicals, enzymes, bacteriophages, metals and organometallic complexes, organic and inorganic catalysts, prosthetic groups, chemiluminescent reactants, and fluorescent molecules.

Where the labeled conjugate in the bound-species is essentially indistinguishable in the presence of the labeled conjugate in the free-species by the means used to monitor the label, as in the case of radioimmunoassay, the bound-species and the free-species must be physically separated in order to complete the assay. This type of assay is referred to in the art as "heterogeneous". Where the bound-species and free-species forms of the labeled conjugate can be distinguished in the presence of each other, a "homogeneous" format can be followed and the separation step avoided.

The present invention relates to immunoassay methods and reagent systems for the quantitative or qualitative determination of an analyte in a test sample in which labeled antibodies are employed.

## 2. *DESCRIPTION OF THE PRIOR ART*

Labeled antibodies have been used in heterogeneous immunoassays employing a wide variety of labeling substances, among them being fluorescent labels [Coons *et al, J. Immunol. 45*:159(1942)], radiolabels [Miles and Hales, *Nature 219*:186(1968)], enzyme labels [U.S. Patent No. 3,654,090], and the like. See *Principles of Competitive Protein-Binding Assays*, ed. Odell and Daughaday, J.B. Lippincott, Phila. (1972) pp. 260 *et seq.* for a general review. In spite of significant inherent advantages in the use of labeled antibodies in direct immunoassay techniques compared to competitive techniques employing labeled analyte conjugates, only limited use has been made of labeled antibodies in the field of homogeneous immunoassays.

U.S. Pat. No. 4,233,402 and Litman *et al, Anal. Biochem. 106*:223(1980) describe an enzyme channeling approach to a homogeneous immunoassay. In this technique, two populations of antibodies to the analyte are labeled respectively with two different enzymes where the product of one of the enzymes is a substrate for the other enzyme. When members of the two labeled antibody populations bind to the analyte, the two enzymes are brought into close contact resulting in an increased rate of production of the ultimately detected signal. This technique requires the labeling of two different sets of antibodies with two different enzymes to obtain the two labeled antibody reagents.

A somewhat similar principle is found in the energy transfer homogeneous immunoassay technique described in U.S. Patent No. 3,996,345 and Ullman *et al, J. Biol. Chem. 251*:4172(1976). In this technique, anti-analyte is labeled with one of a fluorescer-quencher pair and a second reagent is formed with the other of such pair conjugated to analyte. Binding of labeled antibody with the labeled analyte conjugate results in a change in fluorescence due to energy transfer quenching. The presence of analyte from a test sample creates competition for this binding and a corresponding decrease in fluorescence quenching. This technique also requires the preparation of two labeled reagents and, as reported by Ullman *et al*, gives an undesirable biphasic response.

Another homogeneous technique reported in the literature employing labeled antibodies involves the labeling with the enzyme phospholipase C [Wei and Rube, *Clin. Chem. 23*:1386(1977)]. Binding of a high molecular weight analyte (IgG) to the enzyme-labeled antibody causes steric inhibition of the catalytic action of phospholipase C on its substrate, normal erythrocytes. The sensitivity and specificity of such a technique is severly restricted by the difficulty of preparing antibodies of uniform binding avidity and specificity, and removing nonspecific proteins from the antibody preparation. Since conventional methods were employed to produce antiserum, substantial amounts of purified analyte were required as an immunizing agent. The antiserum produced by these methods would consist of a disparate collection of antibodies of differing specificity and binding avidities with the analyte. The isolation of analyte specific antibodies required affinity purification on a matrix to which

the analyte was covalently attached. Again, such an approach would be totally unsuitable for an analyte which itself cannot be obtained in sufficient quantity or purity to prepare both an immunogen and an effective affinity adsorbent. Moreover, in disrupting resulting antigen-antibody complexes on the affinity column in order to obtain free antibody for labeling, the stronger binding, more avid antibodies, the ones most desirable for use in the assay, would be the most difficult to release. Consequently it would be expected that the eluted antibody would predominantly be of a lower avidity or perhaps even denatured antibody. Additionally, unless complement is removed from the antiserum prior to affinity purification, complement may bind to antigen-antibody complexes formed on the affinity column and will elute with antibody to become a potential nonspecifically labeled protein to increase the background signal.

In order to overcome the disadvantages of the prior art homogeneous immunoassays that have attempted to employ labeled antibodies, workers in the field have resorted to even more complicated reagent systems involving additional and more complex reagents. Such is evidenced by the enzyme enhancement immunoassay technique offerred by Gibbons *et al*, *Clin. Chem. 27*: 1602(1981). See also U.S. Pat. No. 4,287,300. In this technique again two populations of antibodies are labeled, one set is chemically modified so as to be negatively charged and the other is labeled with an enzyme. The test sample containing analyte is combined with a reagent system comprising the negatively charged antibody, the enzyme-labeled antibody, and a macromolecular positively-charged substrate

complex. Binding of the two differently labeled antibodies to analyte results in an increased rate of reaction due to the localized attraction of the charged reagents.

Other similarly complex approaches proposed for using labeled antibodies in homogeneous immunoassay are described in U.S. Pat. Nos. 4,208,479 and 4,256,834. In these approaches, a label-modifying macromolecule is required in addition to the signal generation system in order that the response of the label be modulated as a function of analyte.

An object of the present invention is to provide a homogeneous immunoassay method employing labeled antibodies which overcomes the disadvantages of the prior art. Such a method will retain the principal advantages of using labeled antibodies compared to using labeled analyte conjugates while overcoming the losses in sensitivity due to presence of nonspecific labeled proteins and providing a greatly simplified assay protocol involving a single binding reagent, labeled antibody.

A current, general review of the application of monoclonal antibodies in clinical immunology is provided in *Clin. Chem.* *27*:1797(1981). The review lists several advantages of using labeled antibodies in immunoassays and speculates on a few potential future applications of labeled monoclonal antibodies to assay systems based on proximal binding of labeled antibody pairs. The suggestion is to substitute labeled monoclonal antibodies for conventional polyvalent antibodies in those known assay systems employing two different populations of labeled antibodies, such as enumerated above, e.g., enzyme channeling and energy transfer systems.

SUMMARY OF THE INVENTION

The present invention provides a homogeneous immunoassay employing a single labeled antibody reagent. A test sample suspected to contain the analyte of interest is combined with a labeled monoclonal antibody preparation in which antibody to the analyte is substantially the only labeled component. Optionally, the test sample is also combined with a macromolecular conjugate comprising epitopes bindable by the labeled monoclonal anti-analyte. The label employed is selected such that a detectable response is measurably different in a qualitative or quantitative sense when the labeled anti-analyte is bound to the analyte compared to when not so bound, or, when the macromolecular conjugate is also present, is measurably different when the labeled anti-analyte is bound to the conjugate, compared to when not so bound or when bound to analyte. The resulting detectable response is therefore a function of the analyte in the test sample. In the absence of the conjugate, as the analyte concentration increases, there is increased binding of labeled anti-analyte and thus increased modulation of the detectable response of the label. When the conjugate is present, binding of labeled anti-analyte to the conjugate modulates the detectable response of the label. As analyte concentration increases, there is less binding of labeled anti-analyte to the conjugate, and thus decreased modulation of the detectable response of the label.

The anti-analyte is an immunologically derived specific binding substance, e.g., an antibody or fragment thereof. In the preferred embodiment, modulation of the label response upon binding of labeled anti-analyte is caused by steric effects. The label will be selected to provide a detectable response upon

interaction with a member of a reagent detection system with access of said member to the label being hindered sterically upon binding of analyte, or the conjugate, as the case may be, to the labeled anti-analyte. Preferred labels include participants in enzyme-catalyzed reactions, such as substrates, coenzymes, prosthetic groups, inhibitors, and enzymes themselves.

A fundamental feature of the present method is the preparation of labeled anti-analyte wherein the anti-analyte is substantially the only labeled component. By having essentially pure labeled anti-analyte, background response due to label coupled to nonspecific material is virtually absent. The application of monoclonal techniques for obtaining anti-analyte provides a novel and highly advantageous method for preparing such pure labeled anti-analyte preparations.

The preparative method of the present invention comprises the initial step of obtaining a monoclonal anti-analyte preparation by somatic cell hybridization. Nonspecific proteins are readily and efficiently removed by subjecting the monoclonal preparation to a separation technique selective for immunoglobulins. Since substantially all immunoglobulins in the preparation will be monoclonal, i.e., chemically identical, the separated immunoglobulin fraction will be substantially free of nonspecific components. The selected labeling substance is then coupled chemically, e.g., by formation of covalent bonds, to anti-analyte in the purified preparation by any conventional protein modification technique.

Several significant analytical and preparative advantages characterize the present immunoassay

method and reagent system. Principal among them is the requirement for only a single labeled antibody reagent. To determine an analyte, the reagent system need consist only of the labeled anti-analyte and the components of any necessary reagent detection system for interaction with the label to generate a detectable assay response. By employing labeled anti-analyte, the necessity of preparing a unique analyte-label conjugate for each different analyte one might desire to determine is eliminated. Usually, in constructing a series of assays for a group of different analytes in accordance with the present invention, one selects a particular type of anti-analyte to be used in all of the separate assays, e.g., an antibody of the IgG class. Antibodies to the various different analytes will have the same fundamental IgG structure for which a single labeling technique can be used in order to prepare the required labeled anti-analyte reagents. This is a significant synthetic advantage in constructing a family of assays.

A further significant advantage of the present invention is the elimination of the need to extensively purify analyte in order to prepare the labeled conjugate. In the prior art techniques employing analyte-label conjugates, pure analyte is required if significant nonspecific labeling is to be avoided. In the present invention, exquisitely specific anti-analyte is inherently obtained by somatic cell hybridization even where relatively impure analyte preparations are used for animal injections to stimulate production of anti-analyte. Furthermore, a related advantage is that relatively small amounts of analyte are required to obtain large amounts of anti-analyte for labeling and use as an assay reagent because analyte is required in preparing the assay reagents only to stimulate the

initial anti-analyte production. Continued anti-analyte production is possible without the need for any further analyte by known monoclonal antibody proliferation methods.

When analyte is an especially scarce or difficult material to obtain, the present invention provides a further highly advantageous method for preparing the labeled anti-analyte. Essentially pure labeled anti-analyte preparations are obtained by chromatography techniques not requiring analyte as an affinity binder. Through the use of monoclonal antibody techniques, anti-analyte preparations are obtained that contain homogeneous immunoglobulin. Nonspecific material can be removed simply by chromatography with immunoglobulin class-selective binders. All of the labeled antibodies will be of equal avidity in contrast to the prior art attempts at a practical and sensitive assay using labeled conventional antibodies. In the prior art technique, all anti-analyte will be labeled without regard to avidity or specificity, serving to further increase background.

## BRIEF DESCRIPTION OF THE DRAWINGS

Fig. 1 provides comparative diagrams of the respective principles of conventional competitive binding homogeneous immunoassays requiring analyte-label conjugates and of the present invention employing labeled anti-analyte.

Figs. 2-4 are graphical representations of results reported in Example 1 for the determination of human IgG in accordance with the present invention.

Fig. 5 is a graphical representation of results reported in Example 2 for the determination of gentamicin in accordance with the present invention.

## DESCRIPTION OF THE PREFERRED EMBODIMENTS

In the context of this disclosure, the following terms shall be defined as follows unless otherwise indicated:

Analyte - the compound, or class of related compounds, whose presence or amount in a test sample is under determination.

Anti-analyte - an immunologically-derived binding substance specific for the analyte, usually consisting of whole antibodies or antibody fragments.

Homogeneous immunoassay - an assay based on the specific binding between analyte and anti-analyte in which the label response is measured without physical separation of the bound-labeled species from the free-labeled species.

Reagent system - a composition, test device or apparatus, test kit, or other physical arrangement, means, or combination of reagents for use in performing the present assay.

*ASSAY PRINCIPLES*

In a preferred embodiment, the present assay is based on modulation of the response of a label conjugated to anti-analyte upon binding of analyte to such labeled anti-analyte. With reference to Fig. 1, homogeneous immunoassays for determining an analyte (An) based on competitive binding employ an analyte-label conjugate (An*) and anti-analyte (Anti-An). An and An* are placed in competition for binding to a limited amount of Anti-An. Complexes of An and An*, respectively, bound by Anti-An are formed leaving some excess unbound An*. The unbound An* expresses some signal which is modified or modulated in the (An*:Anti-An) complex. In contrast, the present assay can be performed by simple addition of labeled anti-analyte (Anti-An*) with resulting complexes of An and Anti-An*, i.e., (An:Anti-An*), expressing a modified or modulated signal. Modification or modulation of the label response can be the result of a wide variety of possible effects due to binding of analyte with the labeled anti-analyte. For instance, the label response can

be the emission of a signal, e.g., an electromagnetic signal, that is modified in some measurable way by the proximity of the analyte or specific groups on the analyte to the label in the resulting complex. One such instance would pertain to the assay of proteinaceous analytes using a photogenic label, e.g., a fluorescer or chemiluminescer. Proper selection of the label will result in quenching or enhancement of the label signal due to proximity effects with chemical groups in the protein analyte which are either common to proteins in general or unique to the specific protein under assay, e.g., groups which absorb the label emission or hydrophobic groups.

A particularly preferred mechanism of modulation of the label response is steric hindrance. Binding of analyte to labeled anti-analyte will invariably create some steric change in the environment of the label. Appropriate selection of conditions will cause such steric effects to be sufficiently great so as to measurably alter the ability of the label to provide its normal response. Preferably, the label is selected to provide the detectable assay response upon interaction, e.g., chemical reaction, with a member of a reagent detection system comprising one or more substances which interact with the label to generate the measured signal. In such assay systems, the mass of the labeled anti-analyte conjugate will be selected to be significantly increased upon binding of the analyte. As the mass of the analyte becomes relatively larger, the steric effects in the environment of the label become more pronounced.

In cases involving steric hindrance, the label itself will preferably be small, e.g., of molecular weight less than 50,000, generally less than 10,000, more usually less than 4,000, and preferably less than 2,000, and the detection system member or members with which it interacts will preferably be significantly larger, e.g., more than 3 times larger, more usually 10 times larger, and preferably 20 to 100 times or more larger than the label. Accordingly, in the most preferable systems with labels having masses of the order of 100 to 2,000 daltons, at least one member of the detection system with which the label must interact to provide the detectable signal will be of the order of 10,000 to 200,000 daltons or greater. Such size relationship between the label and the detection system member increases the probability of a significant steric effect upon binding of analyte with the labeled anti-analyte. Preferred labels therefore are participants in an enzyme-catalyzed reaction, such as enzyme substrates, coenzymes, enzyme prosthetic groups, and enzyme inhibitors, since a wide variety of enzymic reactions are available from which to chose assay components. Many small substrates, coenzymes, and inhibitors are known for enzymes of sufficiently large molecular weight to have the preferred size relationship between label and its interacting detection system member. This applies likewise for prosthetic groups and their corresponding apoenzymes. Similarly, many enzymes are known which have significantly larger substrates or for which artificially large substrates, coenzymes, or inhibitors can be prepared by coupling small substrates, coenzymes, and inhibitors to high molecular weight backbone materials such as water soluble polymers.

The steric effects of analyte/anti-analyte binding are also enhanced by selecting anti-analytes having masses that will be significantly increased by complexation with analyte. In general, the anti-analyte preferably will be smaller than 10 times the mass of the analyte, more usually smaller than the absolute mass of the analyte, and more preferably smaller than 0.25 the mass of the analyte. The mass of the anti-analyte can be decreased by selecting antibodies of an immunoglobulin class which have low molecular weights, e.g., IgG antibodies have molecular weights around 150,000 whereas those of the IgM class have molecular weights around 900,000. Also, one can selectively cleave antibodies to give fragments of lower molecular weight which retain their specific binding affinity for analyte, e.g., various fragments of IgG antibodies can be prepared such as Fab (50,000 daltons), F(ab') (53,000 daltons), and $F(ab')_2$ (106,000 daltons).

Accordingly, for detection of analyte based on steric hindrance of the label upon binding of labeled anti-analyte, it is preferred to work with large analytes, e.g., molecular weights greater than about 10,000, more usually greater than about 50,000, and preferably greater than about 100,000, and to select relatively small anti-analyte types, e.g., whole IgG antibodies or fragments thereof. Likewise, in such systems it is preferred to select relatively small labels and relatively larger detection system members with which the labels interact.

Where the analyte is not sufficiently large to cause steric hindrance of the label in the labeled analyte upon binding great enough to exhibit a significantly measurable change in the detecable response of the label, an additional component can be added to the system to render it analytically useful. Such additional component is a macromolecular conjugate comprising epitopes (i.e., antigenic determinants or binding sites for antibodies) bindable by the labeled monoclonal anti-analyte whereby binding of such conjugate with labeled anti-analyte causes a measurable change in the label response compared to unbound labeled anti-analyte and compared to labeled anti-analyte bound competitively to analyte. This alternate technique is based on competition between analyte and the analyte-analog macromolecular conjugate for binding with labeled monoclonal anti-analyte. The same parameters apply here as in the preferred direct binding technique in regard to size selections of anti-analyte, label, and the interacting detection system member or members. Further, the macromolecular analyte conjugate will preferably be large in relation to anti-analyte in order to increase steric effects. Preferably, the molecular weight of the macromolecular conjugate will be greater than 0.1 times, more usually greater than, and most preferably 4 times greater than the molecular weight of the selected anti-analyte. Relatively speaking, the analyte will be much smaller than the anti-analyte, usually less than 10 times its molecular weight, and generally will be the size of about 5000 daltons or less, preferably less than about 1000 daltons.

In this alternative technique, the macromolecular conjugate can be constructed in a variety of ways to provide a macromolecule having binding sites recognized by the labeled anti-analyte. For example, the analyte itself, or a specific binding analog (i.e., a compound which is structurally analogous to the analyte and which will be recognized and bind with anti-analyte), may be polymerized or cross-linked to form a macromolecular unit. More usually, the conjugate will comprise a high molecular weight material, e.g., a water soluble polymer, to which are attached, usually by covalent bonds, a number of residues which are either the analyte or a binding analog of the analyte. The backbone material will usually have a molecular weight greater than about 10,000, but can vary as desired for assay performance. The coupling of analyte or analyte analog to such macromolecular matrices will be accomplished by techniques available to the ordinary artisan. The support material may be a natural product, modified natural product, or a synthetic material. Useful backbone or support polymers include proteins, polypeptides, polysaccharides, and the like, with specific examples including albumin, dextran, starch, and agarose. The support may be cross-linked or otherwise chemically modified.

*ANALYTE*

The present invention can be applied to the assay of any analyte for which anti-analyte is available or can be prepared. In most cases, the analyte is a peptide, polypeptide, protein, carbohydrate, glycoprotein, steroid, or other organic molecule for which anti-analyte can be prepared. The analyte, in functional terms, is usually selected from the group comprising antigens and antibodies thereto; haptens and antibodies thereto; and hormones, vitamins, metabolites and pharmacological agents, and their binding counterparts. Usually, the analyte is an immunologically-active polypeptide or protein, usually having a molecular weight of between about 1,000 and about 10,000,000, such as an antibody or antigenic polypeptide or protein, or a hapten having a molecular weight of at least about 100, and usually less than about 1,500.

The preferred direct binding technique of the present invention where binding of analyte with labeled anti-analyte yields modulation of the label response is particularly advantageous as applied to the determination of relatively high molecular weight materials such as polypeptides, proteins, polysaccharides, polynucleic acids and the like. A particular advantage is gained where the analyte is an especially scarce material as is the case with many high molecular weight constituents of test samples of analytical interest, e.g., biological samples such as serum, plasma, whole blood, urine, and saliva. The present assay can be constructed with an absolute minimum amount of purified analyte material.

Representative polypeptide analytes are angiotensin I and II, C-peptide, oxytocin, vasopressin, neurophysin, gastrin, secretin, bradykinin, and glucagon.

Representative protein analytes include the classes of protamines, mucoproteins, glycoproteins, globulins, albumins, scleroproteins, phosphoproteins, histones, lipoproteins, chromoproteins, and nucleoproteins. Examples of specific proteins are prealbumin, $\alpha_1$-lipoprotein, human serum albumin, $\alpha_1$-acid glycoprotein, $\alpha_1$-antitrypsin, $\alpha_1$-glycoprotein, transcortin, thyroxine binding globulin, haptoglobin, hemoglobin, myoglobin, ceruloplasmin, $\alpha_2$-lipoprotein, $\alpha_2$-macroglobulin, $\beta$-lipoprotein, erythroprotein, transferin, hemopexin, fibrinogen, the immunoglobulins such as IgG, IgM, IgA, IgD, and IgE, and their fragments, e.g., $F_c$ and $F_{ab}$, complement factors, prolactin, blood clotting factors such as fibrinogen, thrombin and so forth, insulin, melanotropin, somatotropin, thyrotropin, follicle stimulating hormone, leutinizing hormone, chorionic gonadotropin, thyroid stimulating hormone, placental lactogen, intrinsic factor, transcobalamin, serum enzymes such as alkaline phosphatase, lactic dehydrogenase, amylase, lipase, phosphatases, cholinesterase, glutamic oxaloacetic transaminase, glutamic pyruvic transaminase, and uropepsin, endorphins, enkephalins, protamine, tissue antigens, bacterial antigens, and viral antigens such as hepatitis associated antigens (e.g., $HB_sAg$, $HB_cAg$ and $HB_eAg$).

Hapten analytes can likewise be determined, particularly using the alternate competitive technique taught above involving the use of a macromolecular analyte conjugate. Representative hapten analytes include the general classes of drugs, metabolites, hormones, vitamins, and the like organic compounds. Haptenic hormones include the iodothyronines such as thyroxine and triiodothyronine. Vitamins include vitamins A, B, e.g., $B_{12}$, C, D, E and K, folic acid and thiamine. Drugs include antibiotics such as aminoglycosides, e.g., gentamicin, tobramycin, amikacin, sisomicin, kanamycin, and netilmicin, penicillin, tetracycline, terramycin, chloromycetin, and actinomycetin; nucleosides and nucleotides such as adenosine diphosphate (ADP) adenosine triphosphate (ATP), flavin mononucleotide (FMN), flavin adenine dinucleotide (FAD), nicotinamide adenine dinucleotide (NAD) and its phosphate derivative (NADP), thymidine, guanosine and adenosine; prostaglandins; steroids such as the estrogens, e.g., estriol and estradiol, sterogens, androgens, digoxin, digitoxin, and adrenocortical steroids; and others such as phenobarbital, phenytoin, primidone, ethosuximide, carbamazepine, valproate, theophylline, caffeine, propranolol, quinidine, amitriptyline, cortisol, desipramine, disopyramide, doxepin, doxorubicin, nortriptyline, methotrexate, imipramine, lidocaine, procainamide, N-acetylprocainamide, amphetamines, catecholamines, and antihistamines.

*ANTI-ANALYTE*

This component of the present invention can be any immunologically-derived monoclonal binding substance specific or selective for binding the analyte. When in the form of whole monoclonal antibody, anti-analyte can belong to any of the subclasses of the known classes of immunoglobulins, e.g., IgG, IgM, IgE and so forth. Any fragment of any such antibody which retains specific binding affinity for analyte can also be employed, for instance, the fragments of IgG conventionally known as Fab, F(ab'), and $F(ab')_2$. In addition, aggregates, polymers, and conjugates of immunoglobulins or fragments can be used as anti-analyte where appropriate. Such poly(anti-analytes) can be prepared in any available manner so as to maintain specific binding affinity for the analyte. Other forms of anti-analyte can be employed so long as the material selected or prepared has monoclonal origin and has a specific binding affinity for analyte.

The immunoglobulin source for the anti-analyte of the present invention is obtained by somatic cell hydridization techniques, more commonly referred to as monoclonal antibody techniques. Such techniques are now well-known as general tools for preparing chemically homogeneous antibodies [see the general review *Lymphocyte Hybridomas*, ed. Melchers *et al*, Springer-Verlag (New York 1978) and *Methods in Enzymology 73 (Part B)*:3-46(1981)].

In general terms, monoclonal anti-analyte immunoglobulin is produced by fusing lymphocytes which produce such antibody with myeloma cells to form hybridomas, isolating a hybridoma clone which secretes the desired antibody to the analyte of interest, and harvesting

the secreted monoclonal antibody. The lymphocytes involved in the hybridization are commonly spleen cells removed from an animal such as a mouse or rat which has been immunized against the analyte. Preferably, both the lymphocytes and myeloma cells are of murine origin.

Formed hybridomas which secrete the desired antibody are isolated, usually by cloning on culture media selective for the fused cell hybridomas and determining by appropriate methods an isolated hybridoma which secretes the desired antibody. The homogeneity of the hybridoma cell line is thereafter preferably assured by subcloning the hybridomas selected for their desirable antibody secretion. Harvesting of the secreted monoclonal antibodies is performed by conventional techniques. Proliferation of antibodies can be accomplished by culturing hybridomas *in vitro* or *in vivo*, e.g., introducing the hybridoma into an animal such as a mouse and removing antibody-rich ascites fluid.

*LABEL*

Various types of labels are useful in the present assay, the only requirement being that binding of the labeled anti-analyte with analyte modulate its detectable response. As indicated above, various photogenic labels may be used such as fluorescers and chemiluminescers, where binding of labeled anti-analyte produces a perturbation of their light emission. Preferably, the causative element in modulation of label response is steric hindrance. Following are specific examples, without limitation, of various types of labels which can be used in the present method.

*1. Enzyme substrate-label*

In this system, the label is selected so that the labeled anti-analyte conjugate is a substrate for an

enzyme and the ability of the enzyme to act on the substrate-labeled conjugate is affected, either in a positive or negative sense, but usually in an inhibitory fashion, by binding of the labeled conjugate with analyte. Action of the enzyme on the substrate-labeled conjugate produces a product that is distinguishable in some feature, usually a chemical or physical feature such as chemical reactivity in an indicator reaction or such as a photometric character, e.g., fluorescence or light absorption (color). Labels of this type are described in general terms in commonly assigned, copending application Serial No. 894,836, filed April 10, 1978 (corresponding to U.K. Pat. Spec. 1,552,607); and in *Anal. Chem.* *48*:1933(1976), *Anal. Biochem.* *77*:55(1977) and *Clin. Chem.* *23*:1402(1977). In such enzyme substrate-labeled techniques, the labeled conjugate, e.g., a substrate-anti-analyte conjugate, will have the property that it can be acted upon by an enzyme, by cleavage or modification, to produce a product having a detectable property which distinguishes it from the conjugate. For example, the conjugate can be nonfluorescent under assay conditions but upon reaction with enzyme a fluorescent product is produced.

Various fluorogenic substrate-labeled conjugates are evident for use in such techniques. For example, the labeled conjugate may be of the formula:

$$[G\text{-}D\text{-}R]_n Ab$$

wherein G is a cleavable group such as phosphate, carboxylate, sulfate, or glycone, D is a fluorogenic dye moiety which upon removal of G yields a fluorescent product, e.g., D can be umbelliferone, fluorescein, rhodamine, and their derivatives, R is a linking group, Ab is anti-analyte and *n* is the average number of labels

per molecule of anti-analyte, e.g., between 1 and 50. Enzymatic cleavage (e.g., by phosphatase, carboxylase, sulfatase, glycosidase, etc.) of the labeled conjugate is affected by binding of analyte to the anti-analyte Ab portion of the conjugate. See U.S. Pat. No. 4,279,992. A particularly preferred substrate-labeled assay scheme employs a labeled conjugate of the type:

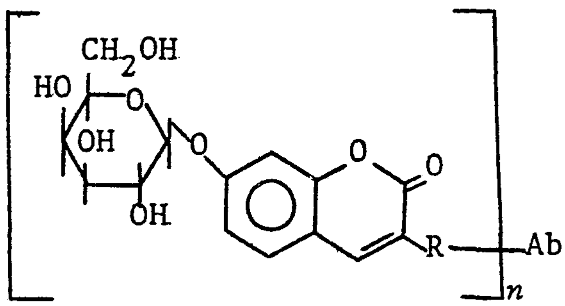

wherein R, Ab, and *n* are as defined above, whereby the ability of the enzyme β-galactosidase to cleave the conjugate yielding a product distinguishable by its fluorescence is inhibited by binding of the conjugate with analyte.

Other useful substrate-labeled conjugates are those of the formula:

$$[D\text{-}R']_n Ab$$

wherein R' is an enzyme cleavable linking group, e.g., phosphate, carboxylate, and the like, Ab and *n* are as defined above, and D is a fluorogenic dye moiety as above which upon cleavage of R releases a fluorescent indicator. A particularly preferred technique employs a labeled conjugate of the type:

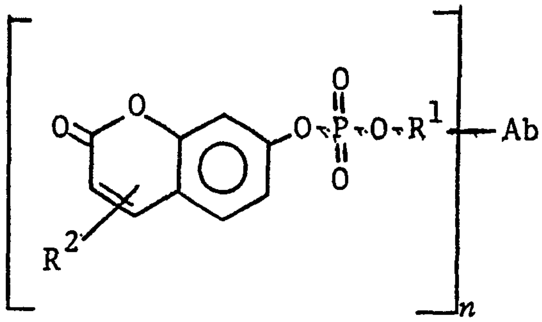

wherein $R^1$ is a bond or chain linking the labeled component Ab to the cleavable phosphate group and $R^2$ is hydrogen or a substituent group such as lower alkyl, e.g., methyl and ethyl, N-alkylamido or N-(hydroxy-substituted lower alkyl)amido, e.g., $-CONH-(CH_2)_m-OH$ wherein *m* = 2-6 (see U.S. Pat. No. 4,273,715). The umbelliferone residue may bear other or additional substituents [see *Anal. Chem. 40*:803 (1968)]. Cleavage by phosphodiesterase is affected by binding of analyte to the anti-analyte Ab portion of the conjugate.

2. *Coenzyme-labels*

The labeled conjugate in this sytem is composed, in its label portion, of a coenzyme-active functionality, and the ability of such coenzyme label to participate in an enzymatic reaction is affected by binding of the labeled anti-analyte conjugate with the analyte. The rate of the resulting enzymatic reaction is measurable by conventional detectant systems to yield an ultimately detectable signal. Labels of this type are described in commonly assigned, copending application Serial No. 894,836, filed April 10, 1978 (corresponding to U.K. Pat. Spec. 1,552,607); and in *Anal. Biochem 72*: 271(1976), *Anal. Biochem 72*:283(1976) and *Anal. Biochem. 76*:95(1976).

*3. Prosthetic Group-labels*

In this system, the label is a prosthetic group of an enzyme, and the ability of a catalytically inactive apoenzyme to combine with the prosthetic group label to form an active enzyme (holoenzyme) is affected by binding of the labeled anti-analyte conjugate with the analyte. Resulting holoenzyme activity is measurably by conventional detectant systems to yield an ultimate detectable signal. Labels of this type are described in commonly owned U.S. Pat. No. 4,238,565. A particularly preferred prosthetic group-labeled assay scheme employs flavin adenine dinucleotide (FAD) as the label and apoglucose oxidase as the apoenzyme. Resulting glucose oxidase activity is measurable by a colorimetric detectant system comprising glucose, peroxidase, and an indicator system which produces a color change in response to hydrogen peroxide. Fluorometric detection of hydrogen peroxide is also possible using an appropriate fluorogenic substrate.

*4. Enzyme modulator-labels*

The labeled conjugate in this system is composed, in its label portion, of an enzyme modulating functionality such as an enzyme inhibitor or stimulator, and the ability of such modulator label to modulate the activity of an enzyme is affected by binding of the labeled anti-analyte conjugate with the analyte. The rate of the resulting enzymatic reaction is measurable by conventional detectant systems to yield an ultimately detectable signal. Labels of this type are described in U.S. Pats. Nos. 4,134,792 and

4,273,866. Particularly preferred is the use of methotrexate as the label with dihydrofolate reductase as the modulated enzyme. Where the label is an enzyme inhibitor, it may interact with the enzyme covalently or noncovalently, and may be a small molecule, e.g., methotrexate, or a large molecule, e.g., antibody to enzyme (see U.S. Pat. No. 4,273,866 and commonly assigned, copending application Serial No. 285,605, filed July 21, 1981).

*5. Enzyme-labels*

In this system, the label is an enzyme and the activity of the enzyme label is affected by binding of the labeled anti-analyte conjugate with the analyte. Resulting enzyme activity is measurable by conventional detectant systems to yield an ultimately detectable signal, e.g., absorption or fluorescence. Labels of this type are described in U.S. Pats. Nos. 3,817,837 and 4,043,872.

*6. Chemically-excited fluorescent-labels*

In this system, the label is again a fluorescer, however, the ability of the fluorescer label to be chemically excited to an energy state at which it fluoresces is affected by binding of the labeled anti-analyte conjugate with the analyte. Chemical excitation of the label is usually accomplished by exposure of the fluorescer label to a high energy compound formed *in situ*. Labels of this type are described in commonly owned U.S. Pat. No. 4,238,195.

*7. Epitope-labels*

In this system, the label comprises an epitope, i.e., an antibody binding site, for a second antibody, i.e., anti-label, or fragment thereof. The ability of anti-label to bind to label in the labeled anti-analyte conjugate is affected by binding of such labeled anti-analyte to the analyte. Several monitoring or detection schemes are possible. In one instance, the epitope label also is a fluorescer whose light emission is altered, e.g., reduced, upon binding with anti-fluorescer. Labeled anti-analyte bound to analyte restricts accessibility of the fluorescer label to the quenching anti-fluorescer (see U.S. Pat. No. 3,998,943). In another approach, an additional detector molecule is used comprising the epitope label coupled to an enzyme. Binding of anti-label to this epitope-enzyme conjugate results in inhibition of enzyme activity. The more anti-label is excluded from binding label on the epitope-labeled anti-analyte by analyte binding, the more anti-label is available to bind to and inhibit enzyme activity of the epitope-enzyme reagent (see U.S. Pat. No. 3,935,074).

*PURIFICATION AND LABELING OF ANTI-ANALYTE*

Once a monoclonal anti-analyte preparation (ascites fluid or tissue culture fluid) has been obtained, a fraction containing essentially only immunoglobulin is separated from the other proteins that may be present. Such separation may be accomplished in any available manner. Preferably, affinity chromatography techniques are applied to this task. As affinity binding partner on the chromatography column can be used anti-(anti-analyte) which usually will be an antibody to the class of immunoglobulin to which the desired anti-analyte belongs, e.g., anti-IgG, or a fragment thereof. While not a preferred method, one can also use analyte as the affinity binding partner. A preferred affinity chromatography technique employs the substance commonly referred to as protein A, a protein excreted by *Staphylococcus aureus* which has the unique property of binding specifically to IgG immunoglobulins [*J. Immunol.* *97*:822(1966) and *Immunol.* *103*:828(1969)]. Protein A is commercially available as the isolated protein or attached to gel particles suitable for use as an affinity chromatography column matrix [Protein A-Sepharose, Pharmacia Fine Chemicals, Piscataway, New Jersey, USA]. The use of protein A to separate anti-analyte from the monoclonal preparation affords a significant advantage to the present invention. The analyte need not be itself purified and attached to a column matrix material to purify anti-analyte. Since the only immunoglobulin present in the monoclonal preparation is anti-analyte one need only apply technique that separate immunoglobulins and it is assured that only anti-analyte will be isolated.

The purified anti-analyte preparation is then labeled according to the particular detection system that is desired. The label, as described above, is coupled to anti-analyte in any available manner. Since the only protein substantially present in the purified anti-analyte preparation is anti-analyte, the labeling substance can be efficiently and specifically attached to anti-analyte therein by reaction under conditions that form covalent bonds between the labeling substance and proteins in general. This ability to use standard protein modification reactions to label anti-analyte without concern for nonspecific labeling of proteins is a further significant advantage of the present invention.

As in conventional labeled conjugates, the label is joined covalently to the component to be labeled, here anti-analyte, by a chemical bond, e.g., a single bond, or by a chain comprising from 1 to 50 atoms, more commonly 1 to 30 atoms, and usually 1 to 20 atoms, excluding hydrogen, principally composed of carbon and heteroatoms selected from nitrogen, oxygen, phosphorous, and sulfur. Conventional linking groups are described at length in the literature. See for example U.S. Pat. Nos. 4,230,797; 4,279,992; 3,817,837; 3,935,074; and 3,996,345. The linking group can be comprised of a side arm group put on the label for the purposes of spacing and/or functionalizing the label for coupling to proteins, and/or the residue from a bifunctional coupling agent used in linking the label or derivatized label to the proteinaceous anti-analyte.

The present labeled anti-analyte conjugates are usually prepared by forming peptide or amide bonds between an amino or carboxyl containing label or label derivative and corresponding carboxyl or amino groups in the anti-analyte protein. Such condensation reactions can be accomplished by conventional peptide bond forming methods such as the carbodiimide reaction [*Science 144*:1344(1974)], the mixed anhydride reaction [Erlanger *et al, Methods in Immunology and Immunochemistry*, ed. Williams and Chase, Academic Press (New York 1967) p. 149], and the acid azide and active ester reactions [Kopple, *Peptides and Amino Acids*, W. A. Benjamin, Inc. (New York 1966)]. For a general review see also *Clin. Chem. 22*:726(1976).

Other well-known methods are also available for coupling the label or a derivative thereof to the anti-analyte. In particular, conventional bifunctional coupling agents can be employed for coupling a label or its derivative, containing a carboxylic acid or amino group, to amino groups in the anti-analyte. For example, amine-amine coupling agents such as bis-isocyanates, bis-imidoesters, and glutaraldehyde [*Immunochem. 6*:53 (1969)] can be used. Also, appropriate coupling reactions are well-known for inserting a bridge group in coupling an amine to a carboxylic acid. Coupling reactions of this type are thoroughly discussed in the literature, for instance in the above-mentioned Kopple monograph and in Lowe & Dean, *Affinity Chromatography*, John Wiley & Sons (New York 1974). Of course, many other ways are available for coupling the label or label derivatives to the various other functionalities in the anti-analyte protein structure such as described in the literature concerning protein modification, including, without limitation, the references cited above,

Means and Feeney, *Chemical Modification of Proteins*, Holden-Day (San Francisco (1971); and Glazer *et al*, *Chemical Modification of Proteins*, Elsevier (New York 1975). Reasonable care should be taken to select procedures which do not denature anti-analyte in order that the labeling step does not lead to the formation of labeled inactive anti-analyte which would raise the background signal.

One or more labels can be attached to an individual anti-analyte molecule and, where the nature of the label allows, *vice versa*. Ordinarily, the anti-analyte will carry between 1 and 30, more usually between 1 and 20, labels per molecule of anti-analyte. While it is desirable to have multiple labels per anti-analyte to enhance sensitivity, too high a labeling density can affect the ability of anti-analyte to effectively bind analyte. In the case where the label is relatively large and polyfunctional, such as an enzyme, multiple anti-analytes can be attached to a single label. Binding to analyte leads to the formation of lattices due to bonding of a single labeled anti-analyte conjugate to multiple analyte molecules, each of which in turn can be bound by labeled anti-analyte, leading to increased interaction, e.g., steric hindrance, at or near the label.

*REACTION MIXTURE AND CONDITIONS*

The test sample to be assayed can be a naturally occurring or artificially formed liquid suspected to contain the analyte, and usually is a biological fluid or a dilution thereof. Biological fluids that can be assayed include serum, plasma, urine, saliva, milk, and amniotic and cerebrospinal fluids.

The binding reaction will in almost all cases be allowed to proceed under mild conditions. The reaction mixture will be in general an aqueous medium with any desirable organic cosolvents being present in minor amounts. The temperature of the reaction will be maintained at a constant level in normal circumstances throughout the incubation period and the measurement step. Temperatures will generally be between 5 and 50°C, more usually between 20 and 40°C. Preferably, the reaction will proceed at room temperature. The pH of the reaction mixture will vary between 5 and 10, more usually between 6 and 9. The concentration of various reagents will depend on the level of analyte expected in the test medium, with such level usually being between $10^{-3}$ and $10^{-12}$M. As in the case of the previously described reaction parameters, selection is primarily based on empirically derived optimization balanced against the preferences and needs of the technician who will ultimately perform assays on a routine basis. None of the parameters therefore is of a critical nature to the present invention, rather they are all within the ordinary skill in the art.

*REAGENT SYSTEM*

The reagent system, i.e., reagent combination or means, of the present invention comprises all of the essential chemical elements required to conduct a desired assay method encompassed by the present invention. The reagent system is presented in a commercially packaged form, as a composition or admixture where the compatibility of the reagents will allow, in a test device configuration, or as a test kit, i.e., a packaged combination of one or more containers holding the necessary reagents. Included in the reagent system are the reagents appropriate for the binding reaction system desired, always requiring a labeled anti-analyte conjugate as defined hereinbefore. Such binding reaction reagents can include, in addition to the labeled conjugate any other necessary or optional reagents for performing the particular assay technique involved. Of course, the reagent system can include other reagents as are known in the art and which may be desirable from a commercial and user standpoint, such as buffers, diluents, standards, and so forth. Also preferred is a test device comprising the reagent system and a solid carrier member incorporated therewith. The various forms of such test device are described in application Serial No. 202,378, filed October 30, 1980, which is incorporated herein by reference.

The present invention will now be illustrated, but is not intended to be limited, by the following examples.

## EXAMPLE 1

### Determination of Human IgG

### A. Purification of Monoclonal Antibody to Human IgG

Mouse ascites fluid containing monoclonal antibody to human IgG ($F_c$ specific; Bethesda Research Laboratories, Rockville, Maryland USA) was purified by affinity chromatography on either human IgG-Sepharose or Staphylococcal Protein A-Sepharose.

(1) Preparation of human IgG-Sepharose

Human IgG-Sepharose was prepared as follows. Sepharose 4B (Pharmacia Fine Chemicals, Piscataway, New Jersey, USA) was washed with distilled water and stored at 4°C overnight. The Sepharose was collected on a coarse glass frit and drained until cracks appeared in the gel. The drained Sepharose 4B was weighed and 20 grams (g) were placed in a plastic beaker with 20 milliliters (ml) of cold water and a magnetic stirring bar. The beaker was placed in a glass crystallizing dish with ice on a magnetic stirrer. A pH electrode and thermometer were inserted, the pH was adjusted to ~ pH 11.5 with 1 drop of 10 N sodium hydroxide (NaOH) and then 8 g of freshly ground cyanogen bromide (CNBr) was added. The pH was maintained at pH 11.0-11.5 by the dropwise addition of 10 N NaOH and the temperature was maintained at 5-10°C by adding ice chips. After 17 minutes the reaction was terminated by decanting the gel onto a coarse glass frit. Some unreacted CNBr remained behind in the beaker.

The gel was washed with ten volumes (200 ml) of cold distilled water followed by 0.1 M sodium carbonate, pH 9.0. The gel was drained until cracks appeared and then it was transferred to a 125 ml Erhlenmeyer flask. Human IgG (150 mg, Research Products Division, Miles Laboratories, Inc., Elkhart, Indiana, USA) dissolved in 10 ml of 0.1 M sodium carbonate buffer, pH 9.0, was added with an additional 5 ml rinse of carbonate buffer. The mixture was placed in the cold room on a see-saw mixer. After 96 hours the gel was decanted onto a coarse glass frit and rinsed with 10 ml of cold distilled water. The filtrate was saved for later analysis to determine the extent of substitution by difference. The gel was resuspended in 50 ml of 0.2 M ethanolamine-HCl, pH 8.0 and incubated at 4°C for 30 minutes on the see-saw mixer. Finally, the gel was decanted on the coarse glass frit and washed with one liter of water, followed by 20 mM Bicine [N,N-*bis*-(2-hydroxyethyl)glycine], pH 8.2, 0.1 M glycine-HCl, pH 3.0 and 20 mM Bicine, pH 8.2.

(2) Purification on human IgG-Sepharose

Mouse ascites fluid containing monoclonal antibody to human IgG was stored frozen at -20°C prior to use. The ascites fluid was applied directly to a 3 ml (1 cm x 3.8 cm) column of human IgG-Sepharose that was equilibrated with 20 mM Bicine, pH 8.2. The column was washed with 15 ml of 20 mM Bicine, pH 8.2. The column was washed with 15 ml of 20 mM Bicine, pH 8.2, then with 15 ml of 20 mM Bicine, pH 8.2, containing 0.1 M sodium chloride (NaCl) and finally with 15 ml of 20 mM Bicine, pH 8.2. The antibody was eluted from the column with 15 ml of 0.1 M glycine-HCl,

pH 2.5, followed by 15 ml of 0.1 M glycine-HCl, pH 2.5, with 20% p-dioxane. Test tubes receiving the low pH eluate contained 100 microliters (µl) of 1 M Bicine, pH 8.5, to increase the pH immediately. The absorbance at 280 namometers ($A_{280}$) of the eluate was monitored and fractions containing the antibody were pooled and concentrated to ~ 1 ml using a Millipore CX-10 immersible ultrafiltration device (molecular weight cut-off $\leq$ 10,000 daltons; Millipore Corp., Bedford, Massachusetts, USA).

The concentrated antibody solution was applied to an 80 ml (1.5 cm x 45 cm) column of Sephadex G-25 (fine; Pharmacia) to remove glycine. Fractions containing the antibody were concentrated using the Millipore CX-10 device.

(3) Purification on Protein A-Sepharose

The ascites fluid (1 ml), stored at -20°C prior to use, was applied to a 4 ml (1 cm x 5 cm) column of Protein A-Sepharose-4B (Pharmacia Fine Chemicals) equilibrated with 20 mM Bicine, pH 8.2, containing 0.04% sodium azide at room temperature. The column was washed with 15 ml of this Bicine buffer, followed by 15 ml of the same Bicine buffer containing 0.1 M NaCl and then 15 ml of Bicine buffer with no NaCl. The antibody was eluted by washing the column with 0.1 M glycine-HCl buffer, pH 3.0. Test tubes receiving this acid eluate contained 100 µl of 1 M Bicine, pH 8.5, to raise the pH immediately. The column was finally washed with 15 ml of Bicine buffer, to return it to pH 8.2. The $A_{280}$ of the 100-drop fractions was monitored and fractions containing the antibody were pooled and concentrated to ~ 1 mL using a Millipore CX-10 immersible ultrafiltration device.

The concentrated antibody solution was chromatographed on Sephadex G25 (fine) to remove glycine. The antibody solution (∿ 1 ml) was applied to an 80 ml (1.5 cm x 45 cm) Sephadex G-25 (fine) column equilibrated with 20 mM Bicine, pH 8.2, containing 0.04% sodium azide at room temperature. The $A_{280}$ of the 100-drop fractions was monitored and fractions containing the antibody were pooled and concentrated, as before, to 2 ml.

B. Labeling of Purified Monoclonal Antibody

The purified monoclonal antibody preparation (2.8 mg/ml) was labeled with β-galactosyl-umbelliferone using the homobifunctional crosslinker dimethyladipimidate (DMA).

Ten micromoles (μmol)(5 mg) of N-6(6-aminohexyl)-7-β-galactosylcoumarin-3-carboxamide(AH-GU) (U.S. Pat. No. 4,259,233) were dissolved in 200 ml $H_2O$ and mixed with an equimolar portion of DMA (10 μmol, 2.5 mg) dissolved in 1 M triethylammonium bicarbonate, pH 9.6 (TEAB). The yellow solution was incubated at room temperature for five minutes, and then 1 ml (2.8 mg) of antibody solution in 20 mM Bicine, pH 8.2, was added and the reaction mixture incubated at room temperature for 60 minutes. The reaction was terminated by the addition of 200 μl of 1 M glycine-NaOH, pH 9.6. The solution was applied to the Protein A-Sepharose column and the labeled antibody was isolated as previously described in part A(3) of this Example.

The labeled antibody was characterized by examining absorbance at 343 nm ($A_{343}$) to quantitate the AH-GU groups present ($\varepsilon^{1mM}_{343} = 21$); absorbance at 400 nm ($A_{400}$) following treatment with excess β-galactosidase to determine the AH-GU groups accessible to the enzyme ($\varepsilon^{1\ mM}_{400} = 35$); and the fluorescence of the hydrolyzed GU-antibody conjugate was examined to determine the extent of quenching.

C. Assay Protocols

(1) Effect of polyethylene glycol on assay reaction

An aliquot (50 µl, 0.013 $A_{343}$ units) of GU-anti-IgG was placed in a cuvette containing 1.25 ml of 20 mM Bicine, pH 8.2, either with or without 6% polyethylene glycol 6000 (Sigma Chemical Co., St. Louis, Missouri, USA) and various amounts (0-200 µl) of normal human IgG (2 mg/ml). The protein concentration was maintained by compensating with bovine serum albumin (BSA). The reaction mixtures were incubated at room temperature for 15 minutes and then 10 µl of 0.61 units/ml β-galactosidase was added. Fluorescence (excitation - 400 nm, emission = 450 nm) was read after an additional 10 and 20 minutes incubation at room temperature. Blanks did not receive β-galactosidase, but were otherwise complete.

(2) IgG assays in the presence of IgA and IgM

An aliquot (50 µl, 0.013 $A_{343}$ units) of labeled antibody to IgG was added to 1.45 ml of 20 mM Bicine, pH 8.2, with 6% polyethylene glycol 6000 and varying amounts of human IgG (or IgA or IgM) (protein concentrations were maintained by compensating decreasing Ig

levels with BSA). The reaction mixtures were incubated at room temperature for 15 minutes and then 10 µl of β-galactosidase (0.61 U/ml) was added. After mixing, the reaction mixtures were incubated a second time for 20 minutes and then the fluorescence was read as above.

D. Results

The labeled monoclonal antibody preparation was found to have 1.35 AH-GU substituents per antibody molecule. When the label was incubated with excess β-galactosidase, 0.61 substituents per antibody molecule (45%) were hydrolyzed and 0.06 substituent per antibody molecule (9.8% of hydrolyzed groups) were detected by fluorescence. The effect of polyethylene glycol 6000 on the inhibition of hydrolysis of the labeled antibody by increasing concentrations of human IgG is shown in Fig. 2 of the drawings. The polyethylene glycol increased the inhibition by 10-20%. The inhibition of GU-anti-IgG hydrolysis by IgG, IgM and IgA are shown in Figs. 3 and 4 of the drawings. The inhibition of GU-anti-IgG reached 50% with 3.8 µg of IgG (0.06 IgG/antibody). The cross-reactivity with IgM (at 20% inhibition) was 15% and with IgA, was 16.4%.

The data establish that an IgG assay can be performed by the method of the present invention. The detectable signal generated by a labeled monoclonal anti-IgG preparation which contains substantially no nonspecific labeled protein is measurably modified upon binding to analyte (IgG).

## EXAMPLE 2

### Determination of Gentamicin

#### A. Purification of Monoclonal Antibody to Gentamicin

Mouse ascites fluid containing monoclonal antibody to gentamicin (Scripps-Miles, Inc., LaJolla, California, USA) was purified by affinity chromatography on Protein A-Sepharose by the method described in Example 1.

#### B. Labeling of Purified Monoclonal Antibody

AH-GU, *supra*, (5 mg, 10 μMol) was dissolved in 200 μl of $H_2O$. Dimethyladipimidate (5 mg, 20 μmol) was dissolved in 400 μl of 1 M Triethylammonium bicarbonate (TEAB), pH 9.6, and 100 μl of this solution was incubated with 100 μl of the AH-GU solution at room temperature for about five minutes. The antibody solution [2 ml, 2.4 mg protein as determined by the Lowry method, *J. Biol. Chem. 193*:265(1951)] was then added, and the reaction mixture incubated at room temperature for about one hour. At this time, 100 μl of 1 M glycine-NaOH, pH 9.6, was added to terminate the reaction. The yellow solution was applied to the Protein A-Sepharose column and the GU-labeled antibody to gentamicin was isolated as described above. The GU-antibody was characterized by measuring $A_{343}$ to determine the concentration of GU substituents present ($\varepsilon_{343}^{1\ mM} = 21$); $A_{400}$ following hydrolysis with β-galactosidase, to determine how many GU substituents could by hydrolyzed ($\varepsilon_{400}^{1\ mM} = 35$); the fluorescence was measured to determine how many of the hydrolyzed GU substituents were quenched.

C. Preparation of Gentamicin-Macromolecular Conjugate

Bovine serum albumin (BSA, 300 mg) was dissolved in 1.5 ml water. An aliquot (1.0 ml) was added to 2.0 ml aqueous solution of gentamicin sulfate (Schering Corp., Bloomfield, New Jersey, USA) having a pH which had been adjusted to about 4.5 by addition of 2 N hydrochloric acid (HCl) or 2 N sodium hydroxide (NaOH) as needed. The pH of the mixture was adjusted similarly to 4.5. Water was added to give a final volume of about 8 ml and the pH again adjusted to 4.5. After cooling in an ice bath for 15 minutes, 600 mg of 1-ethyl-3-(3-dimethylaminopropyl)carbodiimide hydrochloride (Pierce Chemical Co., Rockford, Illinois, USA) was added to the solution and mixed in the ice bath for 2 hours. After overnight incubation at 4°C, the solution was allowed to come to room temperature and then applied to a column (2.5 x 55 cm) of G-25 (fine) Sephadex (Pharmacia Fine Chemicals) equilibrated with 50 mM Bicine-0.1% azide, pH 8.5. Seven (7) milliliter fractions were collected and tested for the presence of BSA-gentamicin conjugate by positive ninhydrin reaction and absorbance at 280 nm. Fractions containing BSA-gentamicin were pooled.

D. Assay Protocol

Labeled monoclonal antibody (10 µl, 0.0014 $A_{343}$ units) was added to 1.48 ml of 20 mM Bicine, pH 8.2, containing 0-80 µg of Gentamicin-BSA or 0-80 µg of Gentamicin-BSA and 0.6 µg of free gentamicin. The reaction mixtures were incubated at room temperature for 15 minutes, and then 10 µl of 0.61 U/ml β-galactosidase was added (except to the blanks) and the reaction mixtures incubated for an additional 20 minutes before the fluorescence was read.

E. Results

The labeled monoclonal antibody preparation was found to have 0.97 AH-GU substituents per antibody molecule. When the label was incubated with excess β-galactosidase, only 0.22 substituents per antibody molecule (23%) were hydrolyzed and 0.023 substituents per antibody molecule (10% of hydrolyzed groups) were detected by fluorescence. The inhibition of GU-anti-gentamicin hydrolysis of gentamicin-BSA and relief of inhibition by free gentamicin are shown in Fig. 5 of the drawings.

The data establish that a gentamicin assay can be performed by the method of the present invention. The detectable signal generated by a labeled monoclonal anti-gentamicin preparation which contains substantially no nonspecific labeled protein is measurably modified upon binding by the gentamicin-macromolecular conjugate. Such signal modulation is relieved by the presence of free gentamicin.

## EXAMPLE 3

### Determination of Human Chorionic Gonadotropin

A. Preparation of Labeled Monoclonal Antibody

Mouse ascites fluid containing monoclonal antibodies to human chorionic gonadotropin (hCG) was purchased from Meloy Laboratories, Inc., Springfield, VA. The antibody was purified by chromatography on Protein A-Sepharose CL-4B (Pharmacia Fine Chemicals), followed by chromatography on Sephadex G-25.

The purified monoclonal antibody was labeled with AH-GU using DMA as in Example 1 above. The labeled antibody was characterized in terms of absorbance, hydrolysis by β-galactosidase, and fluorescence. These analyses showed that the labeled antibody had 0.79 AH-GU labels per antibody molecule; 0.32 of such labels per antibody were hydrolyzed by β-galactosidase; and 0.14 hydrolyzed residues per antibody molecule were detected by fluorescence.

B. Assay Protocol

Aliquots (20 µl, 0.002 $OD_{343}$ units) of labeled monoclonal anti-hCG were added to 1.42 ml volumes of 20 mM Bicine buffer, pH 8.2, containing 0.1% BSA and varying levels of hCG (obtained from Roussel Corp., New York, NY). The solutions were incubated for 15 minutes at room temperature and then 10 µl of β-galactosidase (0.61 U/ml) in 20 mM Bicine, pH 8.2, was added to each. Blanks contained all components except the enzyme. Fluorescence was read after an additional 20 minutes incubation at room temperature.

C. Results

The results are shown in Table A below:

TABLE A

| hCG (units) | Fluorescence Units (corrected for blank) |
|---|---|
| 0 | 3.1 |
| 313 | 2.8 |
| 626 | 2.5 |
| 939 | 2.1 |
| 1565 | 1.5 |

The data demonstrate that an hCG assay can be performed by the present method. Increasing levels of hCG reduce the production of fluorescence from cleavage of the labeled anti-hCG by the enzyme β-galactosidase.

## EXAMPLE 4

### Determination of Human $IgA_1$

A. Preparation of Labeled Monoclonal Antibody

Purified mouse monoclonal antibody to human $IgA_1$ was purchased from Bectin Dickinson, Sunnyvale, CA. The gelatin used to stabilize the antibody was removed by affinity chromatography on Protein A-Sepharose CL-4B, followed by chromatography on Sephadex G-25.

The purified monoclonal antibody was labeled with AH-GU using DMA as in Example 1 above. The labeled antibody was analyzed as in Example 3 above and was found to have 2.15 AH-GU labels per antibody and 0.095 hydrolyzed residues per antibody were detected by fluorescence.

B. Assay Protocol

Aliquots (50 µl, 0.0015 $OD_{343}$ units) of labeled monoclonal anti-human $IgA_1$ were added to 1.25 ml volumes of 20 mM Bicine buffer, pH 8.2, continuing 6% polyethylene glycol 6000 and varying levels of human $IgA_1$ obtained from purification of Cohen Fraction III of normal human plasma protein concentration was maintained by compensating decreasing $IgA_1$ levels with BSA). The solutions were incubated for 15 minutes at room temperature and then 10 µl of 0.61 U/ml β-galactosidase in 20 mM Bicine buffer,

pH 8.2, was added to each. Blanks contained all components except the enzyme. Fluorescence was read after an additional 20 minute incubation at room temperature.

C. Results

The results are shown in Table B below:

TABLE B

| $IgA_1$ (µg) | Fluorescence Units (corrected for blank) |
|---|---|
| 0 | 13.9 |
| 100 | 12.1 |
| 200 | 11.9 |
| 400 | 10.9 |

The data demonstrate that an $IgA_1$ assay can be performed by the present method. Increasing levels of $IgA_1$ reduce the production of fluorescence from cleavage of the labeled anti-$IgA_1$ by the enzyme β-galactosidase.

0088974

WHAT IS CLAIMED IS:

1. A homogeneous immunoassay process for determining an analyte in a test sample, comprising the steps of:

(a) combining said test sample with:

(1) a labeled monoclonal anti-analyte preparation in which said monoclonal anti-analyte is substantially the only labeled component, the label comprised in said labeled monoclonal anti-analyte providing a detectable response which is measurably different when said labeled anti-analyte is bound to said analyte compared to when not so bound, or

(2) a labeled monoclonal anti-analyte preparation, in which preparation said monoclonal anti-analyte is substantially the only labeled component, and a macromolecular conjugate comprising epitopes bindable by said labeled monoclonal anti-analyte, the label comprised in said labeled anti-analyte providing a detectable response that is measurably different when said labeled anti-analyte is bound to said macromolecular conjugate compared to when not so bound, or when bound to said analyte, and

(b) measuring said detectable response as a function of said analyte in said test sample.

0088974

2. The process of Claim 1 wherein said monoclonal anti-analyte preparation comprises whole antibodies.

3. The process of Claim 1 wherein said monoclonal anti-analyte preparation comprises antibody fragments.

4. A homogeneous immunoassay process for determining an analyte in a test sample, comprising the steps of:

(a) combining said test sample with (1) a labeled monoclonal anti-analyte preparation in which said monoclonal anti-analyte is substantially the only labeled component and (2) a reagent detection system for the label comprised in said labeled monoclonal anti-analyte, said label interacting with a member of said detection system to provide a detectable response which is measurably different when said labeled anti-analyte is bound to said analyte compared to when not so bound due to steric hindrance of access of said detection system member to said label, and

(b) measuring said detectable response as a function of said analyte in said test sample.

5. The method of Claim 4 wherein said label is an enzyme substrate, a coenzyme, an enzyme prosthetic group, an enzyme inhibitor, or an enzyme or a participant in an enzyme-catalyzed reaction comprised in said detection system.

6. The method of Claim 4 wherein said analyte has a molecular weight greater than about 10,000.

7. A reagent preparation for use in the homogeneous immunoassay determination of an analyte in a test sample, comprising a labeled monoclonal anti-analyte, the label comprised in said labeled monoclonal anti-analyte providing a detectable response which is measurably different when said labeled anti-analyte is bound to said analyte compared to when not so bound, said monoclonal anti-analyte being substantially the only labeled component in said preparation.

8. A reagent system for the homogeneous immunoassay determination of an analyte in a test sample, comprising (1) a labeled monoclonal anti-analyte preparation in which said monoclonal anti-analyte is substantially the only labeled component, and (2) a macromolecular conjugate comprising epitopes bindable by said labeled monoclonal anti-analyte, the label comprised in said labeled anti-analyte providing a detectable response that is measurably different when said labeled anti-analyte is bound to said macromolecular conjugate compared to when not so bound, or when bound to said analyte.

9. A process for preparing a labeled anti-analyte reagent for use in homogeneous immunoassays to determine an analyte in a test sample, in which preparation said anti-analyte is substantially the only labeled component, comprising the steps of:

(a) obtaining a monoclonal anti-analyte preparation by somatic cell hybridization,

(b) separating a fraction of such preparation containing immunoglobulins from other proteins, and

(c) labeling the protein in said separated immunoglobulin fraction with a material which participates in an enzyme-catalyzed reaction.

10. A labeled anti-analyte preparation comprising a monoclonal anti-analyte labeled with a material which participates in an enzyme-catalyzed reaction, which labeled monoclonal anti-analyte is substantially the only labeled component in said preparation.

I. HOMOGENEOUS IMMUNOASSAY BASED ON COMPETITIVE BINDING

$$An + An^{*} + Anti\text{-}An$$

$$\downarrow$$

$$(An^{*}: Anti\text{-}An) + (An: Anti\text{-}An) + An^{*}$$

[MODIFIED SIGNAL] [SIGNAL]

II. HOMOGENEOUS IMMUNOASSAY WITH LABELED ANTI-ANALYTE

$$An + Anti\text{-}An^{*}$$

$$\downarrow$$

$$(An: Anti\text{-}An^{*}) + Anti\text{-}An^{*}$$

[MODIFIED SIGNAL] [SIGNAL]

FIG. 1

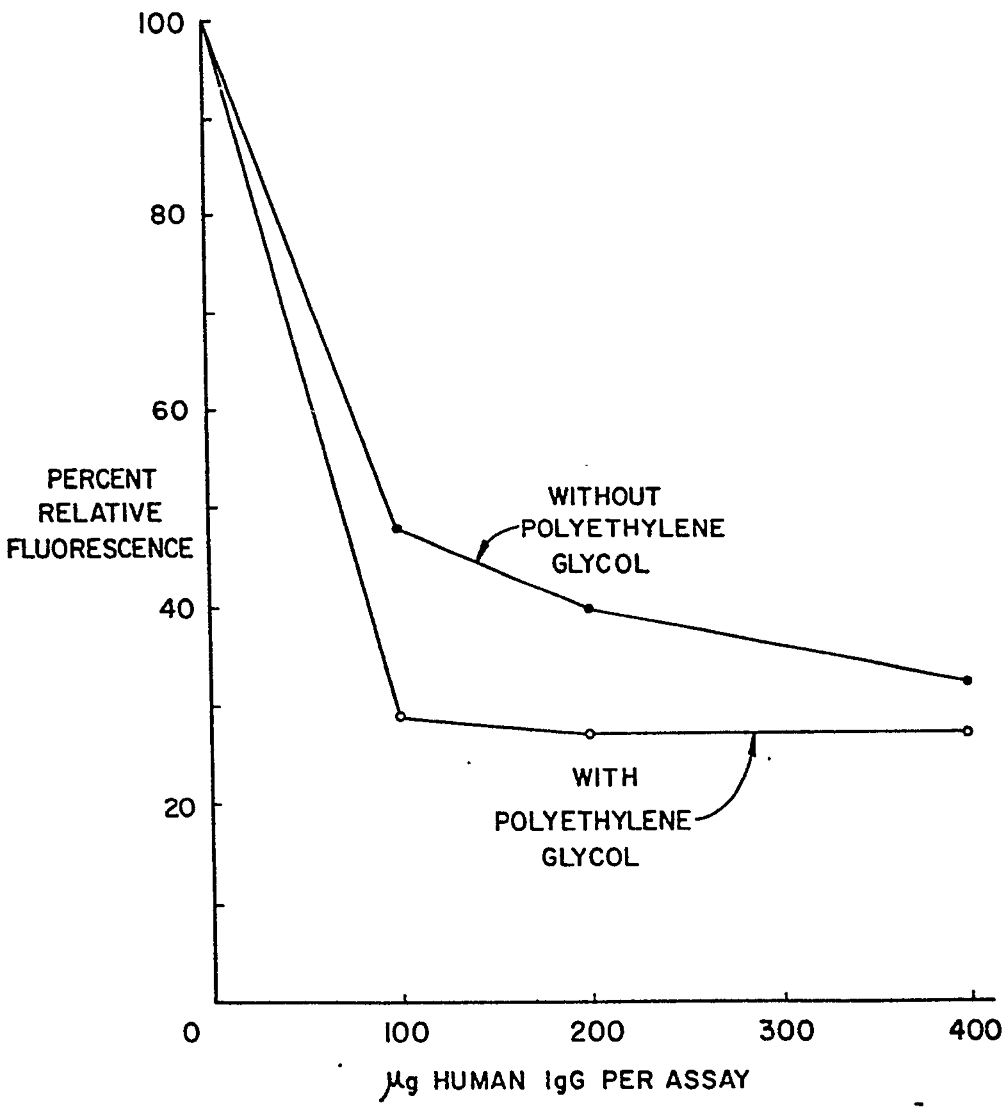
100
80
60
40
20
PERCENT
RELATIVE
FLUORESCENCE
WITHOUT
POLYETHYLENE
GLYCOL
WITH
POLYETHYLENE
GLYCOL
0
100
200
300
400
μg HUMAN IgG PER ASSAY

FIG. 2

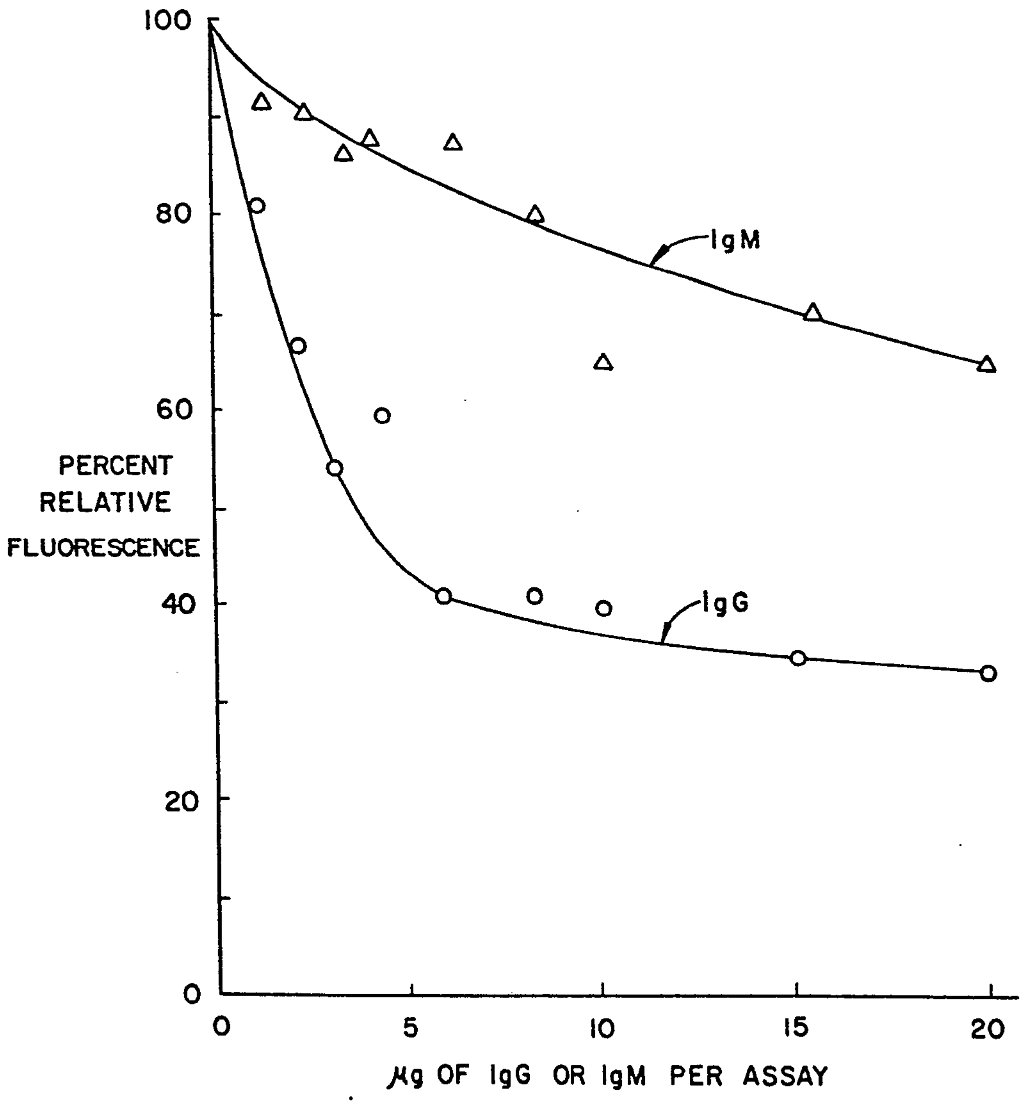
100
80
60
40
20
0
PERCENT
RELATIVE
FLUORESCENCE
IgM
IgG
0
5
10
15
20
μg OF IgG OR IgM PER ASSAY

FIG. 3

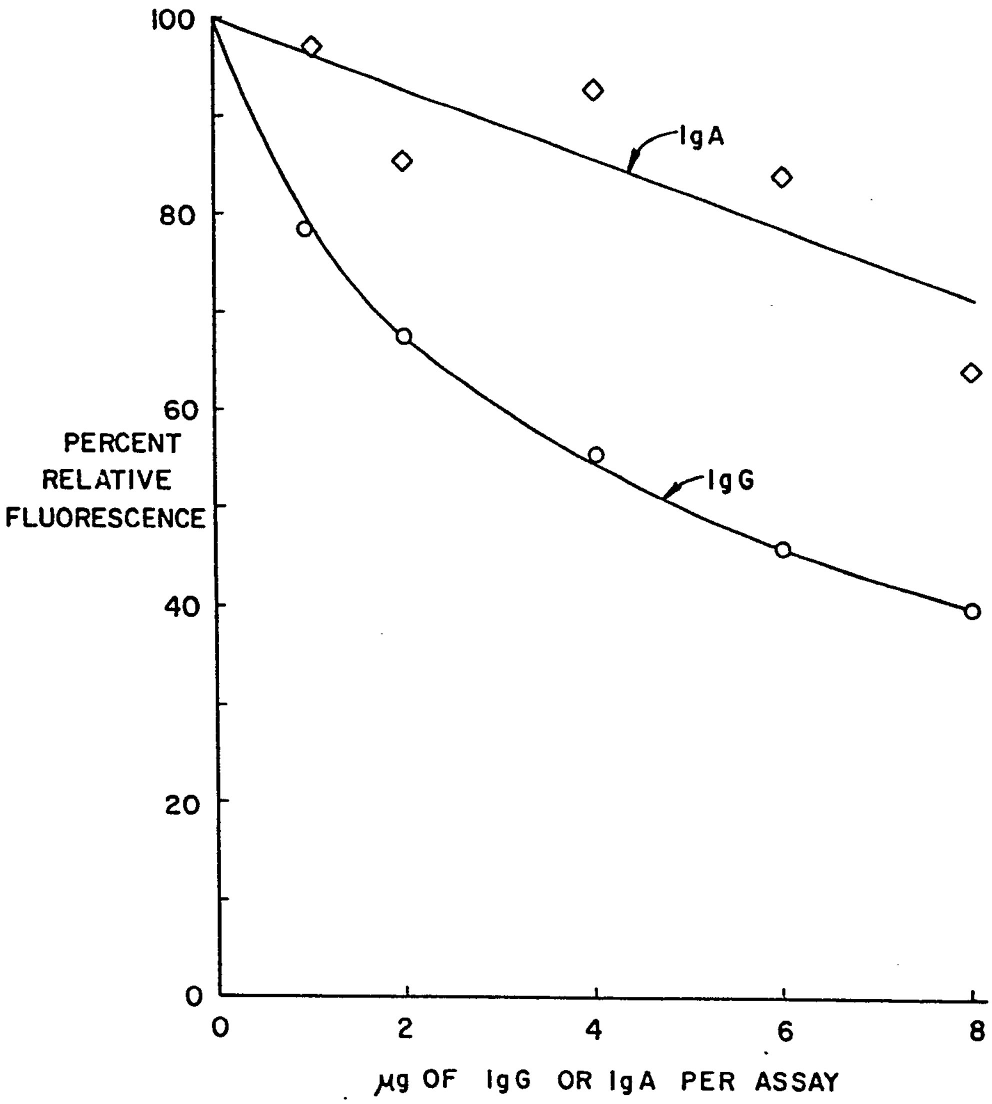

100
80
60
40
20
0
PERCENT
RELATIVE
FLUORESCENCE
IgA
IgG
0
2
4
6
8
µg OF IgG OR IgA PER ASSAY


FIG. 4

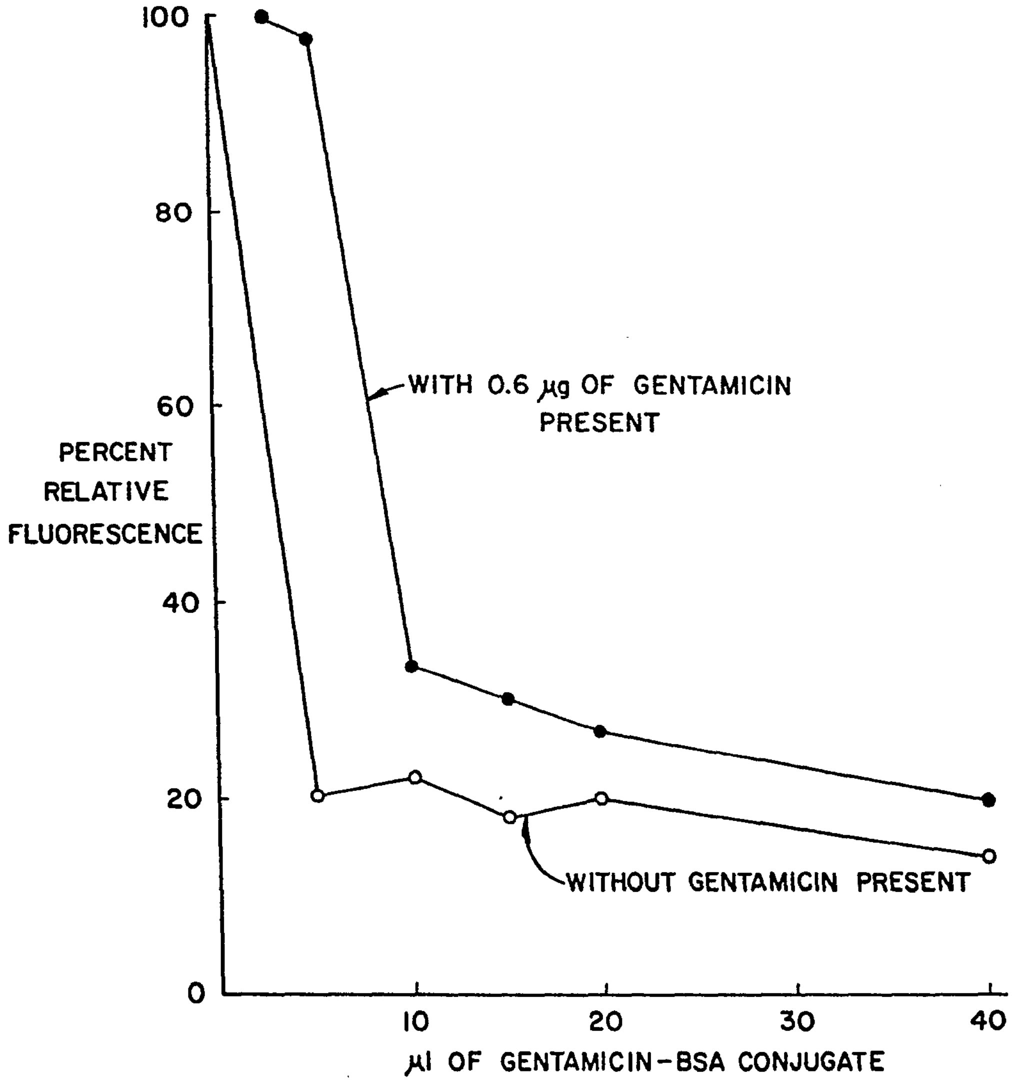
100
80
60
40
20
0
PERCENT
RELATIVE
FLUORESCENCE
WITH 0.6 μg OF GENTAMICIN
PRESENT
WITHOUT GENTAMICIN PRESENT
10
20
30
40
μl OF GENTAMICIN-BSA CONJUGATE

FIG. 5